# EUROPEAN PATENT APPLICATION

(11) **EP 2 573 187 A1**
(43) Date of publication of application: **27.03.2013**
(21) Application number: 11783617.1
(22) Date of filing: 19.05.2011
(51) Int. Cl.: C12P 7/62, C08G 63/00, C12P 1/04

(54) **POLYETHYLENE TEREPHTHALATE-LIKE BIOMASS PLASTIC AND METHOD FOR PRODUCING SAME**

(30) Priority: 19.05.2010 JP 2010128299
(71) Applicant: Seiho Syoji Co., Ltd, Tokyo 103-0001 (JP)
(72) Inventor: SATO, Seiko, Tokyo 121-0011 (JP)
(74) Representative: Vossius & Partner
(86) International application number: PCT/JP2011/061526
(87) International publication number: WO 2011/145683

(57) **Abstract**

The present invention provides a novel biomass plastic. The biomass plastic of the present invention is an alternative to polyethylene terephthalate and is composed of an adhesive polymeric substance. The biomass plastic is obtained by a production method including the steps of: (1) culturing a microorganism mixture composed of Bacillus subtilis, Bacillus pumilus, and Bacillus thuringiensis at 30 to 50°C for 12 hours to 4 days in an environment (medium) including starch and silicic acid or a silicate acceptable in microbial culture; (2) subsequently adding Corynebacterium glutamicum thereto and culturing it at 30 to 50°C for 12 hours to 4 days; and (3) collecting the adhesive polymeric substance from culture obtained.

## Description

### TECHNICAL FIELD

The present invention relates to a novel biomass plastic, a method for producing the biomass plastic, and a molded product of the biomass plastic.

### BACKGROUND ART

Polyethylene terephthalate (PET) is widely used for beverage or food containers, clothing fibers, and the like. PET is produced by chemical reaction of petroleum-derived terephthalic acid and ethylene glycol at high temperatures and under high vacuum.

The present inventor has proposed a biomass plastic alternative to polypropylene (PP) (Patent Document 1). This material is a biodegradable material using, as a binder, an adhesive polymeric substance produced by using Kaoliang starch as a raw material and a combination of three microorganisms of the genus Bacillus. The biomass plastic is produced by the microorganisms and is biodegradable as well as has the feature of using a renewable biomass resource without depending on fossil fuels having the potential to be depleted.

### CITATION LIST

### PATENT DOCUMENT

Patent Document 1: JP2008-137930 A

### SUMMARY OF INVENTION

### TECHNICAL PROBLEM

A more useful material using a renewable biomass resource as a raw material is expected to be proposed. A material that is transparent and relatively highly resistant to heat like conventional PET and can be recycled is particularly desirable.

### SOLUTION PROBLEM

The present inventor has been studying various biodegradable plastics and biomass plastics. As a result of the studies, the present inventor has found that a biomass plastic having the same properties as conventional PET can be obtained by using starch as a culture raw material, mixing and adding three microorganisms of the genus Bacillus thereto and culturing them, and further adding a glutamic acid-producing bacterium of the genus Corynebacterium (Corynebacterium glutamicum), which is one of actinomycetes, and culturing it. The present invention has been thus accomplished. The present invention provides:
[1] A biomass plastic alternative to polyethylene terephthalate, the biomass plastic comprising a polymeric substance, and the polymeric substance being obtained by a production method comprising the steps of:
   (1) culturing a microorganism mixture comprising Bacillus subtilis, Bacillus pumilus, and Bacillus thuringiensis at 30°C to 50°C for 12 hours to 4 days in a medium comprising starch, and silicic acid or a silicate acceptable in microbial culture;
   (2) subsequently adding Corynebacterium glutamicum thereto and culturing it at 30°C to 50°C for 12 hours to 4 days; and
   (3) collecting the polymeric substance from culture obtained.
[2] The biomass plastic according to aspect [1], which has the following IR spectrum: 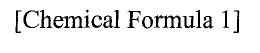 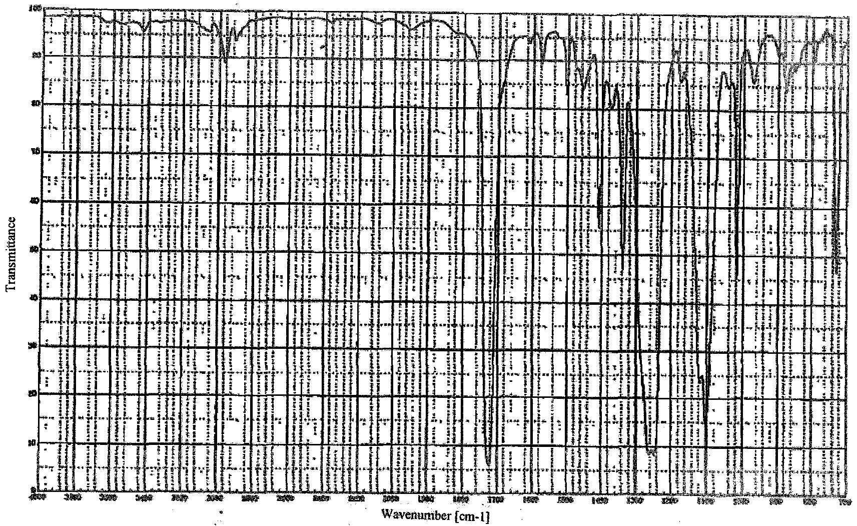
[3] The biomass plastic according to aspect [1] or [2], wherein the starch is derived from one or more plants selected from the group consisting of plants of the family Poaceae (preferably, Kaoliang).
[4] A product obtained by molding the biomass plastic according to any one of aspects [1] to [3].
[5] The product according to aspect [4], which is a beverage or food container, a film, granule, powder, a magnetic tape substrate, or a clothing fiber or clothing.
[6] A method for producing a biomass plastic alternative to polyethylene terephthalate, the biomass plastic comprising a polymeric substance, the method comprising the steps of:
   (1) culturing a microorganism mixture comprising Bacillus subtilis, Bacillus pumilus, and Bacillus thuringiensis at 30 to 50°C for 12 hours to 4 days in an environment (medium) comprising starch and silicic acid or a silicate acceptable in microbial culture;
   (2) subsequently adding Corynebacterium glutamicum thereto and culturing it at 30 to 50°C for 12 hours to 4 days; and
   (3) collecting the polymeric substance from culture obtained.
[7] The method according to aspect [6], wherein the starch is derived from one or more plants selected from the group consisting of plants of the family Poaceae (preferably, Kaoliang).

### ADVANTAGEOUS EFFECTS OF INVENTION

Since a plant-derived raw material can be used in the biomass plastic of the present invention, the biomass plastic does not increase carbon dioxide gas in the atmosphere, that is, the biomass plastic is carbon-neutral and contributes to reduction in use of exhaustible fossil fuels (prevention of global warming).

### BRIEF DESCRIPTION OF DRAWINGS

Fig. 1 shows the IR spectrum of a biomass plastic produced by the present invention.
Fig. 2 shows the IR spectrum of a conventional PET.

### DESCRIPTION OF EMBODIMENTS

The present invention relates to the production of a novel biomass plastic from a specific culture raw material by using microorganisms of the genus Bacillus and actinomycetes.

### (Raw culture materials)

The present invention uses starch as a culture raw material. In the present invention, various plant-derived starches are applicable. Examples of the plants that produce starches applicable to the present invention are as follows:
Plants of the family Poaceae: Kaoliang (Scientific name: Sorghum bicolor; also called Takakibi, Morokoshi, or sorghum), millet, foxtail millet, Japanese barnyard millet, finger millet, pearl millet, teff, kodora millet (kodo millet), wild rice, rice (e.g., sativa-types (Japonica-, Javanica-, Indica-types), glaberrima-types, NERICA), corn (sugar cane), other cereal grains (barley, wheat, rye, Avena fatua, Avena sativa (oat), pearl barley);
Pulse: soybean, adzuki bean, mung bean, black-eyed pea, common bean, lima bean, peanut, green pea, broad bean, lentil (renzu mame), garbanzo bean, lentil (hentou), scarlet runner bean, black gram, moth bean, teriver bean, rice bean, hyacinth bean, horse gram, Bambara bean, geocarpa grand bean, pigeon pea, sword bean, jack bean, grass pea, cluster bean, winged bean, velvet bean, carob, lupinus, tamarind;
Pseudocereals: buckwheat, duck wheat, amaranth, quinoa

The present invention uses starch obtained preferably from plants of the family Poaceae, more preferably from plants belonging to the genus Sorghum, still more preferably from Kaoliang. Kaoliang is excellent in that it can grow even under relatively severe conditions that do not allow rice, wheat, and the like to grow, and according to the present inventor's studies, the biomass plastic of the present invention obtained by using Kaoliang starch as a raw material has been found to be also excellent in physical properties.

When starch is used as a culture raw material in the present invention, starch itself may be used. Alternatively, the fruits or seeds of the foregoing plants may be used if relatively less starch-containing portions such as bran layers (skin and/or aleurone layer) are removed from them as needed before being ground; briefly, the fruits or seeds may be used in the form of a cereal powder rich in starch.

Starch processed (modified) to change its molecular structure to be in a net-like form also may be used for the present invention. Examples of the processed starch include oxidized starch, esterified starch, etherified starch, cross-linked starch, and the like.

In the present invention, additional culture raw materials to be used are silicic acid and/or a silicate acceptable in microbial culture. Examples of the silicate acceptable in microbial culture include magnesium silicate, calcium silicate, sodium aluminosilicate, and aluminum calcium silicate.

In the present invention, one or more of silicic acid or silicates acceptable in microbial culture can be used. As the silicic acid or silicates acceptable in microbial culture, natural substances such as powder of coral fossils (also called coral shell fossils) and shell powder can be used in the present invention. Coral fossils consist primarily of calcium silicate and contain elements such as magnesium, potassium, iron, zinc, phosphorus, and sulfur, in an easily absorbable form.

Microbial spore formation needs silicic acid, and an element such as magnesium accelerates microbial growth rate. Thus, adding silicic acid and/or a silicate acceptable in microbial culture (e.g., a magnesium salt) to the culture system can promote appropriate culturing.

In the present invention, another nutrient source such as ground fruit pulp of jujube may be added to the culture system.

### (Microorganisms)

In the present invention, the initial step is using a microorganism mixture of at least two selected from microorganisms belonging to the genus Bacillus, more specifically, Bacillus subtilis, Bacillus pumilus, and Bacillus thuringiensis. A mixture of three microorganisms of Bacillus subtilis, Bacillus pumilus, and Bacillus thuringiensis is preferably used.

Microorganisms belonging to the genus Bacillus are resident bacteria spreading in the soil or the air, and by making use of their property of forming spores, one skilled in the art can separate the hay bacillus from the natural environment.

The mix ratio of the microorganisms can be adjusted appropriately by one skilled in the art. The adjustment of the mix ratio enables the adjustment of the properties of the biomass plastics produced. When the three microorganisms, Bacillus subtilis, Bacillus pumilus, and Bacillus thuringiensis, are used, an advantageous example of the mix ratio is 3:(3 to 7):(0.5 to 4), and the ratio is preferably 3:(4 to 6):(1 to 3), more preferably 3:(4.5 to 5.5):(1.5 to 2.5). It is to be noted that the microbial ratio as referred to in the present invention means the weight ratio, unless otherwise specified. It is deemed that the weight ratio is about the same as the bacterial count ratio.

In the present invention, the respective existing strains of Bacillus subtilis, Bacillus pumilus, and Bacillus thuringiensis can be appropriately used, but the strains with high productivity of proteins can be particularly advantageously used.

The culture temperature and time can be appropriately determined by one skilled in the art with reference to common conditions for culturing microorganisms of the genus Bacillus. The culture temperature and time are typically 30°C to 50°C and 12 hours to 4 days.

The amount of the microorganisms added to a culture medium can be appropriately determined by one skilled in the art with reference to common conditions for culturing microorganisms of the genus Bacillus. Relative to a culture medium, the amount is typically about 5 wt. % to about 20 wt. % of a culture obtained by fully growing the microorganisms obtained from a culture stock by pre-culturing them in test tubes or the like.

The culture conditions as referred to herein are preferably conditions effective in producing proteins.

In the present invention, the next step is using a glutamic acid-producing bacterium of the genus Corynebacterium, namely, Corynebacterium glutamicum. One skilled in the art can obtain this bacterium from the soil, and the bacterium is also commercially available. In the present invention, an existing strain of Corynebacterium glutamicum can be appropriately used.

The culture temperature and time can be appropriately determined by one skilled in the art with reference to common conditions for culturing microorganisms of the genus Corynebacterium. The culture temperature and time are typically 30°C to 50°C and 12 hours to 4 days.

The amount of the microorganism added to a culture medium can be appropriately determined by one skilled in the art with reference to common conditions for culturing Corynebacterium glutamicum. Relative to a culture medium, the amount is typically about 5 wt. % to about 20 wt. % of a culture obtained by fully growing the microorganism obtained from a culture stock by pre-culturing it in test tubes or the like.

As the culture proceeds, an intended polymeric substance accumulates in the bacterial body. It is deemed that Corynebacterium glutamicum produces the polymeric substance having a benzene ring, by using the proteins produced by the microorganisms of the genus Bacillus.

In this process, the shikimate pathway, which is a reaction pathway for biosynthesis of aromatic amino acids (tyrosine, phenylalanine, and tryptophan), is deemed to be used.

The intended polymeric substance can be separated from cellular residues by using the specific gravity (density) of the polymeric substance (it can be precipitated as gel in the culture because of its high specific gravity). The obtained polymeric substance can be washed and dried, if needed, to thereby prepare a powdery raw material for the biomass plastic. The density of the biomass plastic of the present invention is at least 1.25 g/cm³, more particularly at least 1.28 g/cm³, still more particularly at least 1.31 g/cm³.

### (Biomass plastic)

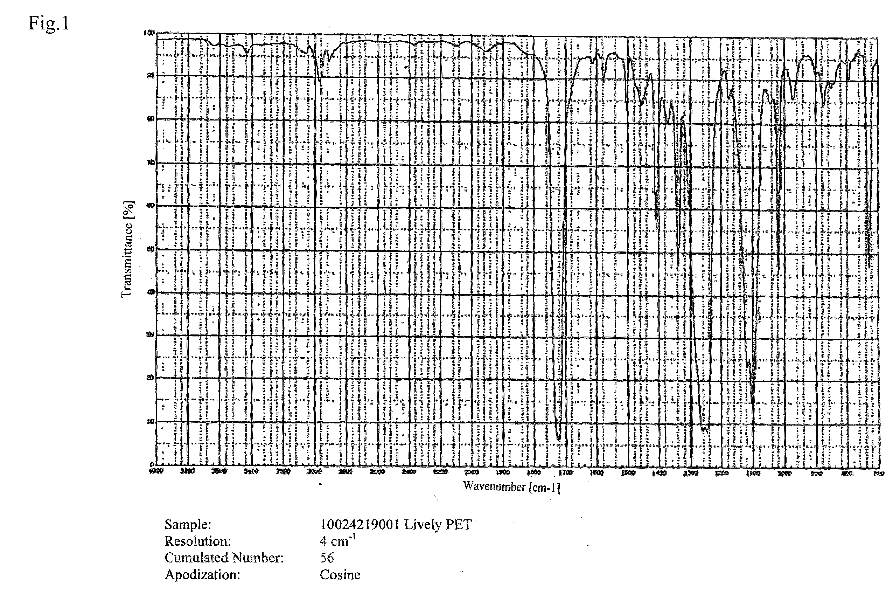

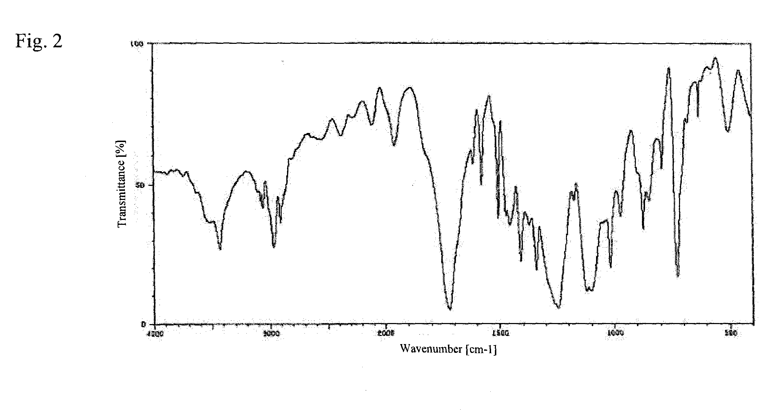

The infrared absorption spectrum of one example of the inventive biomass plastic is shown in Fig. 1. The spectrum is approximate to that of a conventional petroleum-based PET (Fig. 2, provided by the National Agriculture and Food Research Organization, an independent administrative agency). In view of the spectrum, the structural formula of the biomass plastic of the present invention is assumed to contain the structure shown below.

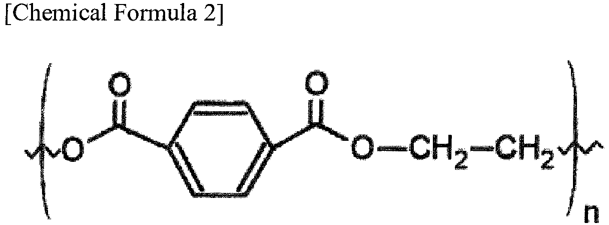

Unless otherwise specified, the biomass plastic alternative to PET, as referred to in the present invention, means a plastic that is distinguishable from petroleum-based PET and characterized by using biomass as a raw material and having a benzene ring on the skeleton of the polymeric structure, thereby acquiring an IR spectrum similar to that of PET.

The inventive biomass plastic alternative to PET can be distinguished from petroleum-based PET.

It is deemed that another distinction between the inventive biomass plastic alternative to PET and petroleum-based PET is that the biomass plastic contains no or less antimony than petroleum-based PET. From petroleum-based PET, a minute amount of antimony is sometimes detected because of antimony-based catalysts used in polymerization. In some cases, the biomass plastic of the present invention contains a very minute amount of antimony derived from coral fossils, shell powder, or the like when they are used in culture; however, the amount is deemed to be lower than in the case of petroleum-based PET.

### (Production of articles such as films and containers of biomass plastic)

The biomass plastic of the present invention can be molded into containers and the like by means for molding known petroleum-based PET in the same manner as in the case of the petroleum-based PET. As a molding method, for example, a stretch blow molding means can be used to form containers.

### (Characteristics of biomass plastic containers)

The biomass plastic containers produced in the present invention have the same properties as petroleum-based PET, and besides the properties, blow-molded biomass plastic containers, in particular, are glossy and superior in quality.

It is to be noted that the biomass plastic described above is generally supplied to the market as a biomass plastic material in the same forms as conventional PET, such as chips, films, plates, and cylindrical bodies.

Further, biomass plastics produced by a method for producing the biomass plastic according to Claim 1, or molded products using these recycled materials provide any of materials such as containers (e.g., bottles), films, granules, powders, magnetic tape substrates, clothing fibers, and clothing.

It is deemed that the biomass plastic of the present invention can replace PET synthesized from conventional fossil resources, and that since the biomass plastic has relatively high heat-resistance like PET, it can be recycled.

Since the biomass plastic of the present invention is produced by the microorganisms, its biodegradation rate is deemed to be high in the natural environment, like existing polyesters produced by microorganisms.

The present invention is described below more specifically with reference to examples, but the examples are not intended to limit the technical scope of the present invention.

### EXAMPLE 1

### (Production of biomass plastic)

To an environment (medium) using 1 kg of Kaoliang starch, ground fruit pulp of jujube, and about 100 g of magnesium silicate (coral fossil powder or shell powder) and containing 10 to 15 wt. % of water, Bacillus subtilis, Bacillus pumilus, and Bacillus thuringiensis were added at a ratio of approximately 3:5:2 to make a total volume of about 10 wt. % (bacteria or florae formed from a mixture of these microorganisms are sometimes referred to as "lively PET bacteria"). These microorganisms were cultured for a while at about 45°C.

Corynebacterium glutamicum was added to this culture and cultured at about 45°C.

At about 36 hours after start of the culture, an intended polymeric substance was recovered. Specifically, it was recovered as a gel-like precipitate by repeating the operations to vibrate the culture minutely several times, since vibrating the culture minutely enables floating of cellular residues having low specific gravities (densities) and meanwhile, enables precipitation of the intended polymeric substance having a high specific gravity. The gel-like polymeric substance could be dried and ground to powder.

### (Identification of biomass plastic)

The obtained biomass plastic raw material powder was processed into sheet or pellet and subjected to an infrared absorption spectrum analysis (IR analysis; transmission microscopy by the Japan Food Research Laboratories). The result is shown in Fig. 1. The IR spectrum of the biomass plastic of the present invention was found to be approximate to the IR spectrum of a petroleum-based PET shown by Fig. 2 (the PET is a product of Mitsubishi Plastics, Inc.; the IR spectrum was provided by the National Agriculture and Food Research Organization, an independent administrative agency).

More specifically, in the both spectra:
1. Absorptions are observed at around 1700 cm⁻¹. They are based on C=O atom groups. The very strong absorptions at around 1700 cm⁻¹, in particular, each indicate the presence of a substance having a benzene ring.
2. Fine absorptions are observed at around 3000 cm⁻¹. They are based on CH atom groups.
3. Very strong absorptions are shown at both around 1250 cm⁻¹ and around 730 cm⁻¹.
4. Approximate absorptions are observed at around 1250 cm⁻¹ or below.

Thus, in light of the similarities described above, the biomass plastic obtained in Example 1 and the petroleum-based PET are assumed to be approximate in structure.

### EXAMPLE 2

The physical tests described below were conducted for the biomass plastic obtained in Example 1 shown above (hereinafter, the biomass plastic is referred to as "lively PET").

### (Test 1)

For a total of three types, lively PET (unprocessed) and two types made transparent by processing at 300°C (heat-resistant lively PETs I and II), the following tests were conducted: IZOD impact test (notched, JIS K7770), flexural strength/flexural modulus (JIS K7770), tensile fracture stress/elongation at break (JIS K7161), density (JIS K7112), and VICAT softening point. The results are as follows:

**[Table 1]**

| Test Items | Lively PET | Heat-resistant Lively PET I | Heat-resistant Lively PET II |
|---|---|---|---|
| IZOD impact strength (kgf•cm/cm) | 4.5 | 6 | 6.7 |
| Flexural strength (kgf/cm²) | 915 | 723 | 718 |
| Flexural modulus (kgf/cm²) | 24,300 | 22,800 | 21,400 |
| Maximum tensile stress (kgf/cm²) | 556 | 567 | 535 |
| Elongation at break (%) | Necking was observed. | Necking was observed. | Necking was observed. |
| Density (g/cm³) | 1.34 | 1.34 | 1.34 |
| Intrinsic viscosity | 0.72±0.015 | 0.76±0.015 | 0.8±0.015 |
| VICAT softening point (°C) | 79 (3.2 mm) | 81 (3.2 mm) | 81 (3.2 mm) |

The results on all of the test items revealed that the three types were as useful as or more useful than petroleum-based PET.

### (Test 2)

Next, the "heat-resistant lively PET I" shown above was used to measure the following items:

**[Table 2]**

| Items | Unit | Value | Test Method |
|---|---|---|---|
| Intrinsic viscosity | dl/g | 0.800±0.015 | ASTM-D-4603 |
| Color (L) | | >82 | ColorQuest XE |
| Color (Lb) | | <1.0 | GB17931 |
| Acid value | meq/Kg | MAX 35 | DSC |
| Melting point | °C | 250±3 | GB 17931 |
| Ash | % | 0.01-0.05 | GB17931 |

The results on all of the test items revealed that the lively PET I was equivalent to petroleum-based PET.

### EXAMPLE 3

The biomass plastic raw material obtained in Example 1 which is an alternative to PET was molded into a beverage bottle by the cold parison method (a parison was preformed by injection molding in the first step, and the preform was stretch blow molded in the second step). As a result, the obtained bottle was superior in surface gloss to petroleum-based PET bottles.

It is deemed that the biomass plastic of the present invention or recycled materials thereof can be used to replace most of articles that have been able to be produced from conventional petroleum-based PET, such as containers (e.g., bottles), films, granules, powders, magnetic tape substrates, clothing fibers, and clothing, in addition to the bottle described above.

### EXAMPLE 4

The same procedure as in Example 1 was performed to attempt to produce biomass plastics except that wheat starch, corn starch, potato starch, cassava starch, or bran was used. As a result, comparable biomass plastics alternative to PET could be obtained from all of the starches.

### INDUSTRIAL APPLICABILITY

The biomass plastic obtained by the present invention is useful as a plant-derived biomass plastic that is an alternative to conventional petroleum-based PET.

## Claims

1. A biomass plastic alternative to polyethylene terephthalate, the biomass plastic comprising a polymeric substance, and the polymeric substance being obtained by a production method comprising the steps of:
(1) culturing a microorganism mixture comprising Bacillus subtilis, Bacillus pumilus, and Bacillus thuringiensis at 30°C to 50°C for 12 hours to 4 days in a medium comprising starch, and silicic acid or a silicate acceptable in microbial culture;
(2) subsequently adding Corynebacterium glutamicum thereto and culturing it at 30°C to 50°C for 12 hours to 4 days; and
(3) collecting the polymeric substance from culture obtained.

2. The biomass plastic according to claim 1, which has the following IR spectrum:

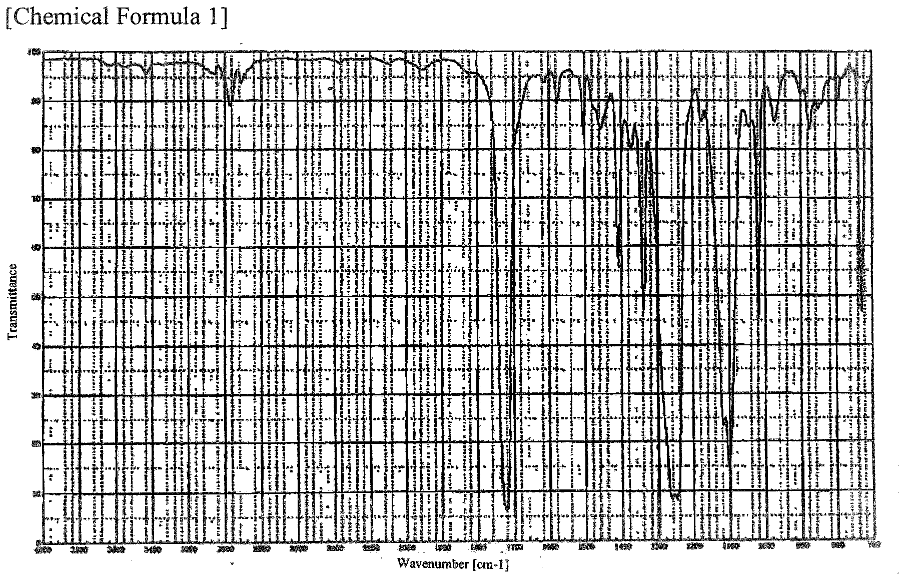

3. The biomass plastic according to claim 1 or 2, wherein the starch is derived from one or more plants selected from the group consisting of plants of the family Poaceae (preferably, Kaoliang).

4. A product obtained by molding the biomass plastic according to any one of claims 1 to 3.

5. The product according to claim 4, which is a beverage or food container, a film, granule, powder, a magnetic tape substrate, or a clothing fiber or clothing.

6. A method for producing a biomass plastic alternative to polyethylene terephthalate, the biomass plastic comprising an adhesive polymeric substance, the method comprising the steps of:
(1) culturing a microorganism mixture comprising Bacillus subtilis, Bacillus pumilus, and Bacillus thuringiensis at 30°C to 50°C for 12 hours to 4 days in a medium comprising starch, and silicic acid or a silicate acceptable in microbial culture;
(2) subsequently adding Corynebacterium glutamicum thereto and culturing it at 30°C to 50°C for 12 hours to 4 days; and
(3) collecting the adhesive polymeric substance from culture obtained.

7. The method according to claim 6, wherein the starch is derived from one or more plants selected from the group consisting of plants of the family Poaceae (preferably, Kaoliang).
